(19) Europäisches Patentamt / European Patent Office / Office européen des brevets

(11) **EP 3 532 163 B1**

(12) **EUROPEAN PATENT SPECIFICATION**

(45) Date of publication and mention
of the grant of the patent:
**15.04.2020 Bulletin 2020/16**

(51) Int Cl.:
*A61N 5/10* *(2006.01)*      *A61B 18/18* *(2006.01)*
*A61N 7/02* *(2006.01)*

(21) Application number: **17791023.9**

(22) Date of filing: **19.10.2017**

(86) International application number:
**PCT/EP2017/076661**

(87) International publication number:
**WO 2018/077709 (03.05.2018 Gazette 2018/18)**

(54) **GRAPHICAL USER INTERFACE FOR ITERATIVE TREATMENT PLANNING**

GRAPHISCHE BENUTZERSCHNITTSTELLE FÜR ITERATIVE BEHANDLUNGSPLANUNG

INTERFACE UTILISATEUR GRAPHIQUE POUR LA PLANIFICATION D'UN TRAITEMENT ITÉRATIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2016 EP 16196215**

(43) Date of publication of application:
**04.09.2019 Bulletin 2019/36**

(73) Proprietor: **Koninklijke Philips N.V.**
**5656 AG Eindhoven (NL)**

(72) Inventors:
• **ISOLA, Alfonso, Agatino**
**5656 AE Eindhoven (NL)**
• **HAUTVAST, Guillaume, Leopold, Theodorus, Frederik**
**5656 AE Eindhoven (NL)**
• **SENDEN, Dave**
**5656 AE Eindhoven (NL)**

(74) Representative: **Groenendaal, Greetje**
**Philips International B.V.**
**Intellectual Property & Standards**
**High Tech Campus 5**
**5656 AE Eindhoven (NL)**

(56) References cited:
WO-A1-2015/083035      US-A1- 2007 201 614
US-A1- 2014 303 423      US-A1- 2015 141 733

## Description

FIELD OF THE INVENTION

The invention generally relates to a user-guided iterative planning of an ablation therapy treatment. More specifically, the invention relates to a system, a method and a computer program for generating a treatment plan for an ablation therapy treatment of a target structure within a treatment region.

BACKGROUND OF THE INVENTION

In ablation therapy, target structures, such as tumors, within patients' bodies are treated by means radioactive or electromagnetic radiation or ultrasound waves in order to control growth of or kill cancer cells. At the same time, the treatment is delivered in such a way that the radiation or thermal dose delivered to surrounding healthy structures, which are usually also referred to as organs at risk, is as low as possible.

One exemplary ablation therapy procedure is the so called temporary brachytherapy in which an applicator is used to place one or more radioactive radiation source(s) within the treatment region for a defined short time interval (usually referred to as dwell time) in order to apply a defined radiation dose particularly to the tumor cells. Further examples of ablation therapy procedures comprise high intensity focused ultrasound (HIFU), radio frequency (RF) and microwave treatments and laser ablation.

The treatment parameters for controlling the ablation therapy treatment are defined in a treatment plan, which is generated in a planning system. In order to determine the treatment plan, a so-called inverse planning procedure may be carried out by the planning system. In such a procedure, the target structure and surrounding organs at risk are identified and treatment goals are specified. Such treatment goals include objectives which may specify requirements for the dose delivered to certain regions of the patient, which should be fulfilled, and/or constraints for the doses delivered to certain regions, which must be fulfilled. Then, an optimization process is carried out to find the treatment plan which fulfills the specified treatment goals.

According to one approach for finding the final treatment plan, an operator-guided iterative optimization procedure is carried out in the planning system. In this procedure, an automatic optimization of the treatment plan is made in several optimization cycles and after each optimization cycle the operator of the planning system (typically a physician) may review the treatment plan as calculated in the respective cycle in order to check whether he/she is satisfied with the dose distribution resulting from this treatment plan. If this is not the case, the operator may make modifications to the optimization problem to achieve a desired dose distribution, and the next automatic optimization of the treatment plan may be carried out on the basis of the modified optimization problem.

The automatic optimization of the treatment plan involves solving an optimization problem, which is formulated on the basis of the objectives and constraints. In a typical planning system, the optimization problem corresponds to the minimization of a cost function which is a weighted sum of individual objective functions, where each individual objective function represents one objective. In addition, it is ensured that the constraints are satisfied. Typical objective functions relate to a minimum dose to be delivered to a certain region of the target structure and to a maximum dose to be delivered to an organ at risk, where the corresponding objective functions are configured such that they have a (global) minimum when these dose requirements are fulfilled.

In order to adapt the treatment plan generated in one optimization cycle in such a system, the dose requirements themselves can generally not be modified, since these requirements are usually set to correspond to the desired dose distribution already at the beginning of the optimization procedure, i.e. prior to the first optimization cycle. Instead, the user typically modifies the weights of the individual objective functions in the cost function. For example, if the user determines that a too high radiation or thermal dose is delivered to an organ at risk, he/she may increase the weight of the individual objective function representing a maximum dose requirement for the relevant organ at risk.

US 2007/0201614 discloses a system and a procedure for optimizing dose delivery of radiation therapy. In order to determine suitable weights of the beamlets of the radiation beam, an overall objective function is minimized which comprises terms relating to all relevant organs and contours. These terms are included in the objective function using importance coefficients. Firstly, the importance coefficients are set to default values according to previous experience and may be altered later as part of an optimization process. Upon having calculated beamlet weights by minimizing the objective function, corresponding dose distribution maps are generated and the user is given the opportunity to indicate whether the planned dose distribution is satisfactory. If this is not the case, the user is prompted to change the importance parameters and a new beamlet weights are determined on the basis of the changed importance parameters.

By modifying the weights or importance parameters assigned to the terms of the individual objective functions, the user can only indirectly influence the dose distribution calculated in the next optimization cycle. Therefore, a dose distribution which is close to the desired dose distribution can usually only be achieved by iterative modifications in a trial and error approach. This is often very time-consuming and may also lead to unsatisfactory results.

## SUMMARY OF THE INVENTION

Before this background, it is an object of the present invention to allow for an easier and more intuitive modification of the optimization problem to be solved in order to generate the treatment plan.

In a first aspect, the invention suggests a system for generating a treatment plan for an ablation therapy treatment of a target structure within a treatment region. The system is configured for treatment plan generation on the basis of optimization objectives and/or constraints representing treatment goals. Further, the system comprises a planning unit which is configured to (i) visualize to a user a dose distribution in the treatment region, the dose distribution corresponding to a first treatment plan generated on the basis of first objectives and/or constraints, (ii) receive a first user input specifying a dose value within the dose distribution and a second user input specifying at least one position in the treatment region as a target position for the dose value, (iii) determine second objectives and/or constraints on the basis of the fist objectives and/or constraints and the first and second user inputs, and (iv) to generate a second treatment plan on the basis of the second objectives and/or constraints.

When reviewing a dose distribution resulting from a treatment plan, the user typically compares dose values of different regions of the dose distribution with each other in order to judge whether or not the dose distribution is satisfying. By specifying a dose value within the dose distribution and at least one position in the treatment region as a target position for the dose value, the user can effectively transfer a dose value from one region to another region. Hence, the user can directly adapt the dose distribution to his/her findings in his/her judgment process by means of the first and second user inputs. Moreover, since second objectives and/or constraints are determined particularly on the basis of the user inputs and a second treatment plan is generated on the basis of the second objectives and/or constraints, the treatment plan can automatically be adapted in accordance with the manipulations of the dose distribution by the user. Hence, it is possible for the user to easily and intuitively control the generation of an adapted treatment plan.

Depending on the applied ablation therapy modality, the dose distribution may particularly correspond to a distribution of a radiation dose or a thermal dose applied to the treatment region in the ablation therapy treatment. Moreover, the treatment parameters specified in the treatment plan may vary depending on the applied ablation therapy modality and may in each case specify the variable treatment parameters relevant in the applied ablation therapy modality.

In one embodiment of the invention, the ablation therapy treatment particularly comprises a temporal brachytherapy treatment. In this case, the treatment plan may specify a dwell time for the temporal brachytherapy treatment. Moreover, the dose distribution may correspond to the radiation dose delivered during the brachytherapy treatment by means of one or radioactive radiation source(s).

In one embodiment of the invention, the planning unit is configured to provide a graphical user interface for graphically visualizing the dose distribution in an image of the treatment region. This allows for an especially easy inspection of the dose distribution and especially easy user inputs. In particular, the user can "transfer" a dose value by means of a drag-and-drop operation including the first and second user inputs using an input means configured for use with a graphical user interface, such as a computer mouse or a trackpad.

In one related embodiment of the invention, the first user input comprises a selection of a position of the treatment region and the specified dose value corresponds to the dose value assigned in accordance with the dose distribution and the second user input comprises a selection of the at least one position of the treatment region. In this regard, the user may select one position of the treatment region as the target position for the specified dose value. Likewise, the user may select a region comprising plural positions as a target region for the specified dose value.

In a further related embodiment of the invention, the first user input comprises a selection of a region comprising plural positions of the treatment region and the specified dose value corresponds to a value derived from the dose values assigned to the plural positions in accordance with the dose distribution and the second user input comprises a selection of the at least one position of the treatment region. Hereby, the user can effectively transfer a dose assigned to a certain source region to a further position or target region. The specified dose value may correspond to a suitable statistical value derived from the dose values assigned to the plural positions in the source region, particularly to an average value of these dose values.

In a further embodiment of the invention, the planning unit is configured to generate the second objectives and/or constraints by adding at least one additional objective and/or constraint to the first objectives and/or constraints and to determine the at least one additional objective and/or constraint on the basis of the first and second user inputs. This allows for a relatively easy implementation of the user inputs into the calculation algorithm for calculating the treatment plan. In particular, this approach for implementing the user inputs involves less complexity compared with other approaches, such as, for example, an adaptation of the first objectives and/or constraints.

In a related embodiment of the invention, the at least one additional objective and/or constraint corresponds to a requirement for the dose delivered to the specified at least one position. Hereby, it can be ensured that the dose distribution resulting from the newly generated treatment plan fulfills the user demands with respect to the specified position or target region.

In this regard, the additional objective or constraint may correspond to a requirement that the dose delivered to the specified at least one position corresponds to the dose value specified by the user. An alternative embodiment of the invention comprises that the planning unit is configured to select the requirement from a maximum dose requirement and a minimum dose requirement on the basis of a comparison between the specified dose value and a dose value assigned to the specified at least one position in accordance with the dose distribution. In particular, the planning unit may be configured to select the maximum dose requirement if the specified dose value is larger than the dose value assigned to the specified at least one position in accordance with the dose distribution. Moreover, the planning unit may particularly be configured to select the minimum dose requirement if the specified dose value is smaller than the dose value assigned to the specified at least one position in accordance with the dose distribution.

In one embodiment of the invention, the planning unit is configured to generate the first treatment plan by minimizing a first cost function containing a weighted sum of objective functions, each objective function corresponding to one of the first objectives. In this embodiment, objectives corresponding to an objective functions having a higher weight are fulfilled with a higher probability. In addition, the cost function may be minimized taking into consideration the constraints in such a way that the constraints are necessarily fulfilled.

In order to generate the second treatment plan, it may generally be preferred to add a further constraint determined on the basis of the user inputs to the first objectives and/or constraints. Then, the second treatment plan may be generated by minimizing the first cost function taking into consideration the additional constraint. Hereby, it can be guaranteed that the user demands are fulfilled.

However, it is likewise possible to add an objective determined on the basis of the user inputs to the first objectives and/or constraints cost function. Therefore, a further embodiment of the invention comprises that the planning unit is configured to generate the second treatment plan by minimizing a second cost function, the second cost function being generated by adding an objective function corresponding to the at least one additional objective determined on the basis of the user inputs to the first cost function.

A related embodiment of the invention comprises that the objective function is added with a weight which is equal to or larger than a maximum of the weights of the objective functions in the first cost function. Hereby, it can be ensured that the newly generated treatment plan fulfills the objective resulting from the user input with a high probability.

In a further aspect, the invention suggests a method for generating a treatment plan for an ablation therapy treatment of a target structure within a treatment region, the treatment plan being generated on the basis of optimization objectives and/or constraints representing treatment goals. The method comprises (i) visualizing to a user a dose distribution in the treatment region, the dose distribution corresponding to a first treatment plan generated on the basis of first objectives and/or constraints, (ii) receiving a first user input specifying a dose value within the dose distribution and a second user input specifying at least one position in the treatment region as a target position for the dose value, (iii) determining second objectives and/or constraints on the basis of the fist objectives and/or constraints and the first and second user inputs, and (iv) generating a second treatment plan on the basis of the second objectives and/or constraints.

In a further aspect, the invention suggests a computer program executable in a processing unit of a system for generating a treatment plan for an ablation therapy treatment, the computer program comprising program code means for causing the processing unit to carry out the method for generating a treatment plan for a patient when the computer program is executed in the processing unit.

It shall be understood that the system of claim 1, the method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically and exemplarily shows an ablation therapy system including a planning unit for generating a treatment plan, and
Fig. 2 schematically and exemplarily shows a visualization of a dose distribution in an image of a treatment region,
Fig. 3 schematically and exemplarily shows an indication of a dose value in the visualization of the dose distribution,
Fig. 4A schematically and exemplarily illustrates a selection of a first position in the visualization of the dose distribution,
Fig. 4B schematically and exemplarily illustrates a transfer of the dose value assigned to the first position to a second position, and
Fig. 5 schematically and exemplarily shows a visualization of a further dose distribution.

DETAILED DESCRIPTION OF EMBODIMENTS

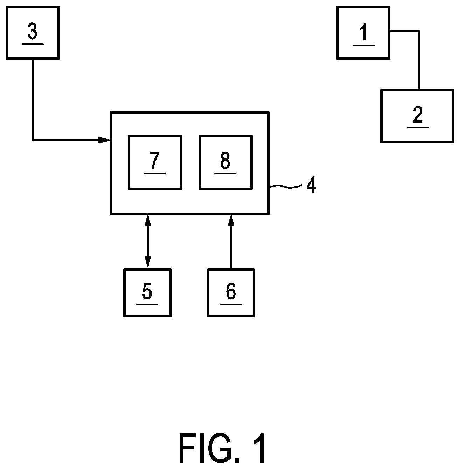

Fig. 1 schematically and exemplarily illustrates an embodiment of a system for delivering ablation therapy treatments to target structures within a human or animal patient body. The target structures may particularly be tumors within certain regions of the body. In one exemplary embodiment, which will be referred to in the following, the system is configured as a temporal brachytherapy system, which may be configured to deliver a high-dose rate (HDR) brachytherapy treatment or another form of temporal brachytherapy treatment. In such a system, the target structure is irradiated by means of one or more radiation source(s), which are temporarily placed in a treatment region in the vicinity of the target structure. This treatment may be delivered once or in plural fractions (i.e. radiation source(s) are placed in the treatment region several times).

In this embodiment, the system comprises an applicator 1 for delivering the radiation source(s) to the treatment region. The radiation source(s) may particularly include radioactive particles emitting ionizing radioactive radiation for treating the target structure. The applicator 1 includes one or more catheter(s) for receiving the radiation source(s). Via the catheters, the radiation source(s) can be delivered to the treatment region and hold at defined positions, which are also referred to as dwell positions, for defined time periods, which are also referred to as dwell times. In the embodiment illustrated in Fig. 1, the radiation source(s) are remotely delivered into the applicator 1 from an afterloader device 2. In further embodiments, the radiation source(s) can likewise be delivered manually into the applicator 1.

Further, the system comprises an imaging device 3 which is configured to acquire images of the treatment region within the patient body. Preferably, the imaging device 3 is configured to generate three-dimensional images of the treatment regions. For this purpose, the imaging device 3 may employ any suitable imaging modality known to a person skilled in the art. Exemplary imaging modalities employed by the imaging device 3 include computed tomography (CT), ultrasound imaging or magnetic resonance imaging (MRI). In principle, it is also possible that the imaging device 3 is configured to acquire two-dimensional images of the treatment region by means of x-ray imaging, ultrasound imaging or another imaging technique. On the basis of the images, the anatomical configuration of the treatment region can be inspected and the relative position of the radiation source(s) and the applicator 1 with respect to the target structure and organs at risk can be determined, when images are acquired while the applicator 1 is positioned in the treatment region.

The treatment is delivered in accordance with a treatment plan, which specifies the relevant treatment parameters particularly including the dwell time and which is generated in a planning unit 4. The planning unit 4 may be configured as a computer device, such as, for example a personal computer, comprising a processing unit which executes a treatment planning software for generating treatment plans for controlling the execution of the radiation therapy treatment. The planning unit 4 comprises a suitable interface for receiving images acquired by means of the imaging device 3. Further, the planning unit 4 comprises or is coupled to a user interface for interacting with a user (which may e.g. be a physician). The user interface may particularly comprise a display unit 5 and an input device 6. The input device 6 may particularly allow for navigating within a graphical user interface provided on the display unit 5. For this purpose, the input device 6 may particularly comprise a pointing device, such as, for example, a computer mouse, a trackpad or a trackball. Likewise, the display unit 5 may comprise a touch-sensitive monitor which also serves as input device 6.

Before commencing an actual radiation treatment in the system, one or more appropriate dwell position(s) in the treatment region is/are determined in a positioning module 7 of the planning unit 4, and the applicator 1 is positioned in the treatment regions such that the radiation source(s) are arranged at the determined dwell position(s) when being inserted into the applicator 1. The dwell position(s) may be determined on the basis of the positions of the target structure and the organs at risk by applying a heuristic determination procedure. Known examples of such a procedure include the so called k-means clustering procedure and the so called centroidal Voronoi tessellation. The positions of the target structure and the organs at risk may be determined using an image of the treatment region acquired by means of the imaging device 3. In the image, the target structure and the organs at risk may be delineated to determine the contours of the target structure and the organs at risk, and the positions may be determined on the basis of the determined contours. The delineation of the target structure and the organs at risk may be made using a manual, semiautomatic or automatic procedure known to the person skilled in the art.

On the basis of the arrangement of the dwell position(s) relative to the target structure and the organs at risk, the treatment plan is determined in a plan module 8 of the planning unit 4 in a way to be described in more detail herein below. The treatment plan particularly defines the dwell time during which the treatment region is irradiated by means of the radiation source(s). Upon having positioned the applicator 1 in the treatment region and upon having determined the treatment plan for the dwell position(s) of the radiation source(s), the radiation source(s) is/are delivered into the applicator 1 and hold in place within the applicator 1 in accordance with the treatment plan.

The treatment plan is generated on the basis of a clinical prescription for the patient, which may particularly specify a radiation dose to be delivered to the target structure during the treatment. In addition, maximum radiation doses to be delivered to the organs at risk may be specified. This may be done in the prescription for the patient and/or in general rules relating to the treatment. Moreover, the treatment plan is generated on the positions of the target structure and the organs at risk determined from an image of the treatment region, which is also referred to as planning image herein below. The planning image may correspond to the aforementioned image on the basis of which the dwell positions are determined. Likewise another image of the treatment region may be used.

On the basis of the treatment goals, a set of objectives and/or constraints is determined and a final treatment plan is generated which at least approximately fulfills the objectives and constraints. For this purpose, an optimization problem is formulated on the basis of the objectives and constraints, and this optimization problem is at least approximately solved in a user-guided iterative optimization procedure comprising several steps. In each step, the plan module 8 automatically calculates a preliminary treatment plan by approximating a solution of the optimization problem. Then, the plan module 8 determines the dose distribution corresponding to this treatment plan and visualizes the dose distribution to the user of the planning unit 4. The user reviews the dose distribution to decide whether he/she is satisfied with the dose distribution or not. If the user is satisfied in one step, the treatment plan calculated in this step is used as the final treatment plan. If the user is not satisfied, the optimization problem is modified in accordance with changes specified by the user as a result of his/her review. Then, the plan module 8 calculates a new preliminary treatment plan in the next step.

Each treatment plan results in a certain dose distribution delivered to the treatment region, where the dose distribution corresponds to the spatial distribution of radiation dose values in the treatment region. Hence, since the planning is made with respect to the image volume of the planning image, the dose distribution allocates a dose value to each voxel of the treatment region.

Objectives correspond to requirements that the dose distribution should fulfill. The possible objectives particularly comprise the delivery of a maximum and minimum radiation dose to specific locations or regions within the treatment region. Minimum dose requirements usually relate to the target structure. So a minimum radiation dose to be delivered to one or more locations or regions of the target structure may particularly be specified. Maximum dose requirements usually relate to the organs at risk. In this regard, a maximum radiation dose to be delivered to one or more locations or regions of the organs at risk may particularly be specified. In addition, further objectives may be defined, such as, for example, the delivery of a uniform dose distribution to a certain region of the treatment volume (which will usually be a region of the target structure).

Constraints generally correspond to the same requirements as the objectives. However, while requirements implemented as objectives do not have to be exactly fulfilled, the dose distribution must no violate requirements implemented as constraints.

In order to automatically generate a treatment plan on the basis of the objectives and constraints specified for a particularly patient, the plan module 8 may minimize a cost function F using a suitable optimization algorithm. The cost function F may comprise a collection of individual objective functions $F^k$, where each individual objective function $F^k$ represents one objective. In one embodiment, the cost function F may particularly correspond to a weighted sum of the objective functions $F^k$, i.e.

$$F(\tau) = \sum_{k=1}^{N} w^k F^k \,,$$

where $\tau$ denotes the set of treatment parameters (i.e. the dwell time in the present example) to be determined and the parameter $w^k$ denotes the weight of the objective function $F^k$. Due to the weighting, objectives having a higher weight are satisfied more likely than objectives having a lower weight, in case such objectives are in conflict with each other. Hence, the weights are selected in accordance with the importance of the objectives with respect to the success of the treatment.

As an example, the objective function representing a maximum/minimum radiation dose for a certain volume V may be given by

$$F^k = \sum_{i \in V} f\left(d_i, d^k\right) \cdot \left[\frac{d_i - d^k}{d^k}\right]^2 \cdot \Delta v_i \,,$$

where $f(d_i, d^k) = H(d_i - d^k)$ in case a maximum dose is specified and $f(d_i, d^k) = H(d^k - d_i)$ in case a minimum dose is specified. $\Delta v_i$ denotes the volume of the voxel i, $d_i = d_i(\tau)$ is the radiation dose delivered to the voxel i when the radiation parameters $\tau$ are used, $d^k$ is the maximum/minimum radiation dose to be delivered to the volume V, and H is the Heaviside step function defined by

$$H(x) = \begin{cases} 0, & x < 0 \\ 1, & x \geq 0 \end{cases}.$$

A constraint may be represented by a function $C(\tau)$ so that the plan module 8 may minimize the aforementioned function $F(\tau)$ and may at the same time ensure that

$C(\tau) \geq 0$ or $C(\tau) = 0$

is fulfilled. For instance, the corresponding function C for a constraint corresponding to a maximum dose requirement may be $C = d^k - d_i$ and the function C for a constraint corresponding to a minimum dose requirement may be $C = d_i - d^k$. In order to (approximately) solve the optimization problem such that the constraints are fulfilled, the known method of Lagrangian multipliers can be applied, for example.

In order to determine the treatment parameters such that the objective function is (approximately) minimized and the constraints are fulfilled, any suitable optimization algorithm may be used by the plan module 8. In one exemplary implementation, the plan module 8 may use the NPSOL algorithm described in P.E. Gill et al., "User's guide for NPSOL 5.0: A Fortran package for nonlinear programming", Technical Report SOL 86-6, Revised 2001. However, any other suitable algorithm known to a person skilled in the art may likewise be used.

In such a way, the plan module 8 may calculate a treatment plan in each step of the user-guided optimization procedure explained above. Moreover, as said above, the optimization problem is modified in accordance with changes specified by the user, when the user is not satisfied with the dose distribution resulting from the generated treatment plan. In this regard, the plan module 8 determines the dose distribution corresponding to the generated treatment plan in each step and visualizes the dose distribution to the user. For this purpose, the planning image may be displayed at the display unit 5 under the control of the plan module 8, and the planning image may be overlaid with a graphical representation of the dose distribution.

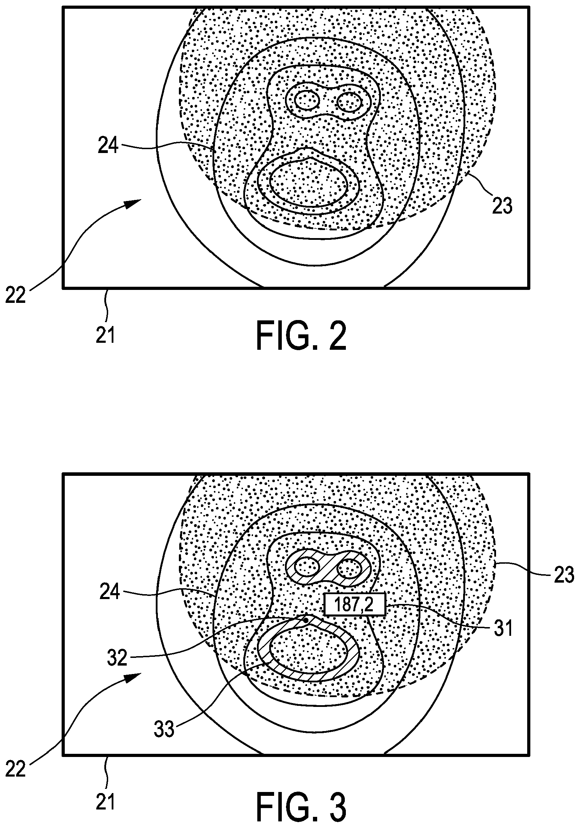

In particular, the dose values corresponding to the dose distribution may be visualized in accordance with predefined dose intervals and the graphical representation may contain isodose curves of the interval boundaries. By way of example, such a graphical representation is illustrated in Figure 2, which shows an image 21 of a treatment region 22 that includes a target structure 23. The image 21 is overlaid with the isodose curves of the boundaries between certain dose intervals, where one isodose curve is provided with the reference numeral 24.

In addition or as an alternative to the isodose curves, the graphical representation of the dose distribution may contain semi-transparent colorings or shadings of regions with dose values from the same interval (which are referred to as isodose regions herein below).

In the graphical user interface comprising the image and the aforementioned representation of the dose distribution, the user can preferably control movements of a cursor by operating the input device 6. In order to review the dose distribution in more detail, the user may move the cursor over the image and the graphical user interface may display the dose values assigned to positions or voxels passed over by the cursor. For this purpose, an indication 31 specifying the dose value may be overlaid over the image as shown in Figure 3, or the dose value may be specified in the graphical user interface next to the image. The figure also shows the cursor, provided with the reference numeral 32, which is positioned at the location to which the indicated dose value is assigned. In addition, the isodose region 33, to which the voxel passed over by the cursor belongs, may optionally be highlighted as likewise shown in figure 3 (in the figure the isodose region is shaded). Hereby, the dose distribution may be further clarified to the user.

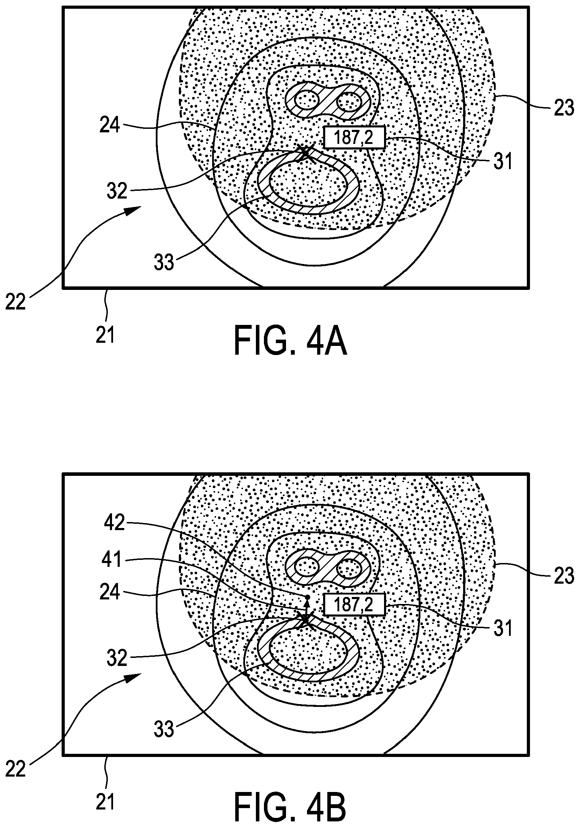

When the user is not satisfied with the dose distribution, he/she can initiate a calculation of a new treatment plan resulting in a modified dose distribution in the graphical user interface. In particular, the user can select a first position or voxel to which a certain dose value is assigned in accordance with the current dose distribution. The selection may be made by navigating the cursor to this location and by performing a predetermined input action, such as, for example, a click on a mouse button, when the cursor is located at the relevant location. Upon the selection of the first position, the assigned dose value may be specified in the graphical user interface, e.g. in the manner explained above, and the selected first position may be highlighted in the graphical user interface. This situation in which the selected position is highlighted and the assigned dose value is specified is exemplarily illustrated in Figure 4A. In this figure, the highlighting is illustrated using a cross. However, the highlighting can be provided in any other suitable way.

By the selection of the first position, the assigned dose value is captured. Now, the user can selected a second position to which the dose value is being assigned as a target dose value for the next optimization step. In Figure 4b, the second position is provided with the reference numeral 42. Thus, the user can transfer the dose value assigned to the first position as a target dose value to the second position. In order to achieve this, the user may navigate the cursor to the second position and then select this position by performing a predetermined input action. In the graphical user interface, the transfer may be visualized e.g. by means of an arrow connecting the first and second position. Such an arrow 41 is exemplarily illustrated in Figure 4b.

In this way, the user can directly and easily manipulate the dose distribution. In particular, the user can transfer a dose value from one position to another. In this regard, the user typically compares dose values of different regions of the dose distribution (rather than checking absolute dose values) in order to judge whether or not the dose distribution is satisfying. By transferring dose values as described above, the user can directly adapt the dose distribution to his/her findings in this judgment process (i.e. the findings "The dose value at the second position is too low/high compared with the dose distribution at the first position"). This allows for an intuitive manipulation of the dose distribution.

In response to the user inputs, the plan module 8 preferably modifies the optimization problem for determining the treatment plan in such a way that the dose value at the second position will approach the specified target dose value (e.g. the value specified by the user) in accordance with the treatment plan calculated in the next optimization step. For this purpose, the plan module 8 adds a corresponding constraint or objective to the set of constraints and objectives used for calculating the treatment plan in the current optimization cycle.

In this regard, the plan module 8 preferably adds a constraint to this set, which corresponds to a maximum or minimum dose requirement for the second position depending on whether the dose value specified by the user is smaller or larger than the current dose value assigned to the second position. This is determined in the plan module 8 by comparing the dose value specified by the user with the current dose value. More specifically, the plan module 8 adds a constraint corresponding to a maximum dose requirement for the second position if the dose value specified by the user is smaller than the current dose value. This means that the plan module 8 adds a constraint function $C = d_f - d_i$ to the list of constraint functions (which have to be equal to or larger then zero), where $d_f$ corresponds to dose value of the second position according to the treatment plan to be calculated and $d_i$ corresponds to the dose value at the first position specified by the user as explained above. In case the dose value specified by the user is larger than the current dose value, the plan module 8 adds a constraint corresponding to a minimum dose requirement for the second position. Hence, the plan module 8 adds a constraint function $C = d_i - d_f$ to the list of constraints functions.

By adding such constraints, the plan module 8 can ensure that the dose value will approach the specified target dose value. In principle, it would also be possible to add a constraint requiring that the dose value of the second position equals the dose value specified by the user. However, such a constraint may result in an optimization problem that cannot be solved by the optimization algorithm. Therefore, the addition of a constraint corresponding to a maximum or minimum dose requirement is preferred.

In an alternative embodiment, the plan module 8 can add an objective corresponding to a maximum or minimum dose requirement to the set of objectives and constraint. In this case, the modifications made by the user may not be realized with some probability. However, the "search space" of possible solutions to the optimization is larger in this case so that the optimization algorithm may produce a better overall solution in some situations.

In this alternative embodiment, the plan module 8 may particularly add an objective corresponding to a maximum dose requirement for the second position, if the dose value specified by the user is smaller than the current dose value. Thus, the plan module adds an objective function of the form

$$F^f = H\left(d_f - d_i\right) \cdot \left[\frac{d_i - d_f}{d_i}\right]^2 \cdot \Delta v$$

to the cost function to be minimized. If the dose value specified by the user is larger than the current dose value, the plan module 8 may particularly add an objective corresponding to a maximum dose requirement for the second position. Thus, the plan module 8 adds to the cost function an objective function of the form

$$F^f = H\left(d_i - d_f\right) \cdot \left[\frac{d_i - d_f}{d_i}\right]^2 \cdot \Delta v .$$

As an alternative, the plan module 8 may add an objective requiring that the dose value of the second position equals the dose value specified by the user. Such an objective could be implemented by adding to the cost function and objective function of the form

$$F^f = \left[\frac{d_i - d_f}{d_i}\right]^2 \cdot \Delta v$$

As in case of the addition of a corresponding constraint, such an objective limits the "space" of possible solutions of the optimization problem. However, since an objective does not necessarily have to be fulfilled, it may still be possible to solve the optimization problem in any case in this embodiment.

The weights of the added objectives in the cost function may be specified by the user. As an alternative, the plan module 8 may automatically select a weight. In particular, the plan module 8 may evaluate the weights of the objective functions already included in the cost function and may selected the largest weight among these weights for the new objective function. Hereby, it is ensured that the added objective will be fulfilled with a high probability.

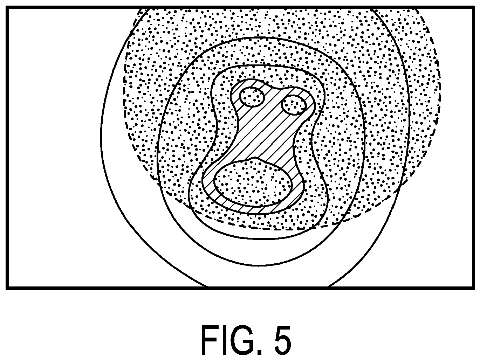

Upon having modified the optimization problem by adding the aforementioned constraint or objective, the optimization procedure proceeds to the next optimization step. In this optimization step, the modified optimization problem is automatically solved in order to generate a new preliminary treatment plan, and the dose distribution corresponding to the new treatment plan is displayed at the display unit 5 as explained above. In order to illustrate the changes of the dose distribution resulting from the modifications made by the user, the isodose region including the previously specified first position may be highlighted as explained above. In the new dose distribution, this isodose region should also include the second position previously specified by the user. This is exemplarily shown in Figure 5, which also illustrates a potential result of the transfer of the dose value illustrated in Figures 4A und 4B.

In the way described above, the user can transfer a dose value from a first position to a particular second position. The same may be done for plural first and second positions in one optimization step, when the user is not satisfied with the dose values of plural second positions. Moreover, the plan module 8 can also be configured to enable the transform of a dose value from a first position to plural second positions forming a contiguous target region. In this case, the constraint or objective added to the set of the constraints and objectives by the plan module relates to all voxels included in the target region In order to specify the target region, any suitable input procedure may be used. For example, an enlarged cursor with a certain shape may be provided to mark and select the target region, or the target region may be manually delineated by moving the cursor along the contour of the region.

Furthermore, it may be possible for the user to specify a source region comprising plural first positions and to transfer a dose value derived from the dose values of the plural first positions to a second position and/or a target region. The source region may be specified in the same manner as described above. The derived dose value may be calculated from the dose values of the plural positions in a suitable way. In particular, the derived dose value may correspond to a suitable statistical value calculated from the dose values of the plural positions. For example, the derived dose value may correspond to a maximum, minimum or an average of the individual dose values. When a target region is specified, it is likewise possible to estimate the mean and the variance of the dose values of the plural positions in the source region. In this case, the mean may correspond to the target dose value and the plan module 8 may add an objective requiring that each position of the target region has a dose value corresponding to the determined mean. Such an objective may be implemented by adding to the cost function a corresponding objective function as explained above with respect to each position in the target region. In addition, the plan module 8 may add a constraint, which requires that the variance of the dose values in the target region does not exceed the variance of the dose values in the source region. Hence, dose values in the target region, which deviate from the mean are generally possible in the dose distribution resulting from the new version of the treatment plan but the variance of the dose values will not exceed that of the dose values in the source region. Hereby, it is possible to generate a dose distribution within the target region which is statistically similar to the dose distribution in the source region.

While the embodiments described above relate to the generation of a treatment plan for a temporal brachytherapy treatment, it is likewise possible to apply the procedures described above to other ablation treatment modalities such as, for example, HIFU, RF and microwave treatments and laser ablation. These treatment modalities differ from the temporal brachytherapy treatment in that a thermal dose distribution is determined in the planning process instead of a radiation dose distribution. Moreover, the treatment parameters are different and may include the time period during which energy is applied to the treatment region as well as the power and the frequency of the radiation or ultrasound beam. Thus, the aforementioned procedures can be applied to these treatment modalities when the relevant treatment parameters are optimized instead of the dwell time and a thermal dose distribution is determined and manipulated by the user instead of a radiation dose distribution.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A system for generating a treatment plan for an ablation therapy treatment of a target structure (23) within a treatment region, the system being configured for treatment plan generation on the basis of optimization objectives and/or constraints representing treatment goals, and the system comprising a planning unit (4) configured to

   - visualize to a user a dose distribution in the treatment region, the dose distribution corresponding to a first treatment plan generated on the basis of first objectives and/or constraints,
   - receive a first user input specifying a dose value within the dose distribution and a second user input specifying at least one position (42) in the treatment region as a target position for the dose value,
   - determine second objectives and/or constraints on the basis of the fist objectives and/or constraints and the first and second user inputs, and
   - generate a second treatment plan on the basis of the second objectives and/or constraints.

2. The system as defined in claim 1, wherein the planning unit (4) is configured to provide a graphical user interface for graphically visualizing the dose distribution in an image (21) of the treatment region.

3. The system as defined in claim 2, wherein the first user input comprises a selection of a position of the treatment region and the specified dose value corresponds to the dose value assigned to the position in accordance with the dose distribution and wherein the second user input comprises a selection of the at least one position (42) of the treatment region.

4. The system as defined in claim 2, wherein the first user input comprises a selection of a region comprising plural positions of the treatment region and the specified dose value corresponds to a value derived from the dose values assigned to the plural positions in accordance with the dose distribution and wherein the second user input comprises a selection of the at least one position (42) of the treatment region.

5. The system as defined in claim 1, wherein the planning unit (4) is configured to generate the second objectives and/or constraints by adding at least one additional objective and/or constraint to the first objectives and constraints and to determine the at least one additional objective and/or constraint on the basis of the first and second user inputs.

6. The system as defined in claim 5, wherein the at least one additional objective and/or constraint corresponds to a requirement for the dose delivered to the specified at least one position (42).

7. The system as defined in claim 6, wherein the planning unit (4) is configured to select the requirement from a maximum dose requirement and a minimum dose requirement on the basis of a comparison between the specified dose value and a dose value assigned to the specified at least one position (42) in accordance with the dose distribution.

8. The system as defined in claim 7, wherein the planning unit (4) is configured to select the maximum dose requirement if the specified dose value is larger than the dose value assigned to the specified at least one position (42) in accordance with the dose distribution.

9. The system as defined in claim 7, wherein the planning unit (4) is configured to select the minimum dose requirement if the specified dose value is smaller than the dose value assigned to the specified at least one position (42) in accordance with the dose distribution.

10. The system as defined in claim 1, wherein the planning unit (4) is configured to generate the first treatment plan by minimizing a first cost function containing a weighted sum of objective functions, each objective function corresponding to one of the first objectives.

11. The system as defined in claims 5 and 10, wherein the planning unit (4) is configured to generate the second treatment plan by minimizing a second cost function, the second cost function being generated by adding an objective function corresponding to the at least one additional objective to the first cost function.

12. The system as defined in claim 11, wherein the objective function is added with a weight which is equal to or larger than a maximum of the weights of the objective functions in the first cost function.

13. The system as defined in claim 1, wherein the ablation therapy treatment comprises a temporal brachytherapy treatment and wherein the treatment plan specifies a dwell time for the temporal brachytherapy treatment.

14. A method for generating a treatment plan for an ablation therapy treatment of a target structure within a treatment region, the treatment plan being generated on the basis of optimization objectives and/or constraints representing treatment goals, the method comprising:

visualizing to a user a dose distribution in the treatment region, the dose distribution corresponding to a first treatment plan generated on the basis of first objectives and/or constraints, receiving a first user input specifying a dose value within the dose distribution and a second user input specifying at least one position (42) in the treatment region as a target position for the dose value, determining second objectives and/or constraints on the basis of the fist objectives and/or constraints and the first and second user inputs, and generating a second treatment plan on the basis of the second objectives and/or constraints.

15. A computer program executable in a processing unit of a system for generating a treatment plan for an ablation therapy treatment, the computer program comprising program code means for causing the processing unit to carry out a method for generating a treatment plan for a patient as defined in claim 14 when the computer program is executed in the processing unit.

**Patentansprüche**

1. System zum Erzeugen eines Behandlungsplans für eine Ablationstherapiebehandlung einer Zielstruktur (23) innerhalb einer Behandlungsregion, wobei das System zum Erzeugen des Behandlungsplans auf der Basis von Optimierungszielsetzungen und/oder Einschränkungen, die Behandlungsziele darstellen, konfiguriert ist, und wobei das System eine Planungseinheit (4) umfasst, die konfiguriert ist zum

- Visualisieren einer Dosisverteilung in der Behandlungsregion für einen Benutzer, wobei die Dosisverteilung einem ersten Behandlungsplan entspricht, der auf der Basis von ersten Zielsetzungen und/oder Einschränkungen erzeugt wurde,
- Empfangen einer ersten Benutzereingabe, die einen Dosiswert innerhalb der Dosisverteilung spezifiziert, und einer zweiten Benutzereingabe, die mindestens eine Position (42) in der Behandlungsregion als eine Zielposition für den Dosiswert spezifiziert,
- Bestimmen von zweiten Zielsetzungen und/oder Einschränkungen auf der Basis der ersten Zielsetzungen und/oder Einschränkungen und der ersten und der zweiten Benutzereingabe, und
- Erzeugen eines zweiten Behandlungsplans auf der Basis der zweiten Zielsetzungen und/oder Einschränkungen.

2. System nach Anspruch 1, wobei die Planungseinheit (4) konfiguriert ist, um eine graphische Benutzeroberfläche zum graphischen Visualisieren der Dosisverteilung in einem Bild (21) der Behandlungsregion bereitzustellen.

3. System nach Anspruch 2, wobei die erste Benutzereingabe eine Auswahl von einer Position der Behandlungsregion umfasst und der spezifizierte Dosiswert dem Dosiswert entspricht, der gemäß der Dosisverteilung der Position zugewiesen ist, und wobei die zweite Benutzereingabe eine Auswahl der mindestens einen Position (42) der Behandlungsregion umfasst.

4. System nach Anspruch 2, wobei die erste Benutzereingabe eine Auswahl einer Region mit mehreren Positionen der Behandlungsregion umfasst und der spezifizierte Dosiswert einem Wert entspricht, der von den Dosiswerten abgeleitet wurde, die gemäß der Dosisverteilung den mehreren Positionen zugewiesen sind, und wobei die zweite Benutzereingabe eine Auswahl der mindestens einen Position (42) der Behandlungsregion umfasst.

5. System nach Anspruch 1, wobei die Planungseinheit (4) konfiguriert ist, um die zweiten Zielsetzungen und/oder Einschränkungen durch Addieren von mindestens einer zusätzlichen Zielsetzung und/oder Einschränkung zu den ersten Zielsetzungen und Einschränkungen zu erzeugen und um die mindestens eine zusätzliche Zielsetzung und/oder Einschränkung auf der Basis der ersten und der zweiten Benutzereingabe zu bestimmen.

6. System nach Anspruch 5, wobei die mindestens eine zusätzliche Zielsetzung und/oder Einschränkung einer Anforderung der an die spezifizierte mindestens eine Position (42) abgegebenen Dosis entspricht.

7. System nach Anspruch 6, wobei die Planungseinheit (4) konfiguriert ist, um die Anforderung aus einer maximalen Dosisanforderung und einer minimalen Dosisanforderung auf der Basis eines Vergleichs zwischen dem spezifizierten Dosiswert und einem Dosiswert, der gemäß der Dosisverteilung der spezifizierten mindestens einen Position (42) zugewiesen ist, auszuwählen.

8. System nach Anspruch 7, wobei die Planungseinheit (4) konfiguriert ist, um die maximale Dosisanforderung auszuwählen, wenn der spezifizierte Dosiswert größer ist als der Dosiswert, der gemäß der Dosisverteilung der spezifizierten mindestens einen Position (42) zugewiesen ist.

9. System nach Anspruch 7, wobei die Planungseinheit (4) konfiguriert ist, um die minimale Dosisanforderung auszuwählen, wenn der spezifizierte Dosiswert kleiner ist als der Dosiswert, der gemäß der Dosisverteilung der spezifizierten mindestens einen Position (42) zugewiesen ist.

10. System nach Anspruch 1, wobei die Planungseinheit (4) konfiguriert ist, um den ersten Behandlungsplan durch Minimieren einer ersten Kostenfunktion zu erzeugen, die eine gewichtete Summe von Zielfunktionen enthält, wobei jede Zielfunktion einer von den ersten Zielsetzungen entspricht.

11. System nach einem der Ansprüche 5 und 10, wobei die Planungseinheit (4) konfiguriert ist, um den zweiten Behandlungsplan durch Minimieren einer zweiten Kostenfunktion zu erzeugen, wobei die zweite Kostenfunktion erzeugt wird, indem eine Zielfunktion, die der mindestens einen zusätzlichen Zielsetzung entspricht, zu der ersten Kostenfunktion addiert wird.

12. System nach Anspruch 11, wobei zu der Zielfunktion ein Gewicht addiert wird, das gleich oder größer ist als ein Maximum der Gewichte der Zielfunktionen in der ersten Kostenfunktion.

13. System nach Anspruch 1, wobei die Ablationstherapiebehandlung eine temporäre Brachytherapiebehandlung umfasst und wobei der Behandlungsplan eine Verweilzeit für die temporäre Brachytherapiebehandlung spezifiziert.

14. Verfahren zum Erzeugen eines Behandlungsplans für eine Ablationstherapiebehandlung einer Zielstruktur innerhalb einer Behandlungsregion, wobei der Behandlungsplan auf der Basis von Optimierungszielsetzungen und/oder Einschränkungen, die Behandlungsziele darstellen, erzeugt wird, wobei das Verfahren Folgendes umfasst:

Visualisieren einer Dosisverteilung in der Behandlungsregion für einen Benutzer, wobei die Dosisverteilung einem ersten Behandlungsplan entspricht, der auf der Basis von ersten Zielsetzungen und/oder Einschränkungen erzeugt wurde,

Empfangen einer ersten Benutzereingabe, die einen Dosiswert innerhalb der Dosisverteilung spezifiziert, und einer zweiten Benutzereingabe, die mindestens eine Position (42) in der Behandlungsregion als eine Zielposition für den Dosiswert spezifiziert,

Bestimmen von zweiten Zielsetzungen und/oder Einschränkungen auf der Basis der ersten Zielsetzungen und/oder Einschränkungen und der ersten und der zweiten Benutzereingabe, und

Erzeugen eines zweiten Behandlungsplans auf der Basis der zweiten Zielsetzungen und/oder Einschränkungen.

15. Computerprogramm, das in einer Verarbeitungseinheit eines Systems zum Erzeugen eines Behandlungsplans für eine Ablationstherapiebehandlung ausführbar ist, wobei das Computerprogramm Programmcodemittel umfasst, um die Verarbeitungseinheit zu veranlassen, ein Verfahren zum Erzeugen eines Behandlungsplans für eine Patienten nach Anspruch 14 durchzuführen, wenn das Computerprogramm in der Verarbeitungseinheit ausgeführt wird.

**Revendications**

1. Système pour générer un plan de traitement pour un traitement de thérapie d'ablation d'une structure cible (23) au sein d'une région de traitement, le système étant configuré pour une génération de plan de traitement sur la base d'objectifs et/ou contraintes d'optimisation représentant des buts de traitement, et le système comprenant une unité de planification (4) configurée pour

- visualiser pour un utilisateur une distribution de dose dans la région de traitement, la distribution de dose correspondant à un premier plan de traitement généré sur la base de premiers objectifs et/ou contraintes,
- recevoir une première entrée d'utilisateur spécifiant une valeur de dose dans la distribution de dose et une seconde entrée d'utilisateur spécifiant au moins une position (42) dans la région de traitement en tant que position cible pour la valeur de dose,
- déterminer des seconds objectifs et/ou contraintes sur la base des premiers objectifs et/ou contraintes et des première et seconde entrées d'utilisateur, et
- générer un second plan de traitement sur la base des seconds objectifs et/ou contraintes.

2. Système selon la revendication 1, dans lequel l'unité de planification (4) est configurée pour fournir une interface utilisateur graphique pour visualiser graphiquement la distribution de dose sur une image (21) de la région de traitement.

3. Système selon la revendication 2, dans lequel la première entrée d'utilisateur comprend une sélection d'une position de la région de traitement et la valeur de dose spécifiée correspond à la valeur de dose attribuée à la position en conformité avec la distribution de dose et dans lequel la seconde entrée d'utilisateur comprend une sélection de l'au moins une position (42) de la région de traitement.

4. Système selon la revendication 2, dans lequel la première entrée d'utilisateur comprend une sélection d'une région comprenant plusieurs positions de la région de traitement et la valeur de dose spécifiée correspond à une valeur dérivée des valeurs de dose attribuées aux plusieurs positions en conformité avec la distribution de dose et dans lequel la seconde entrée d'utilisateur comprend une sélection de l'au moins une position (42) de la région de traitement.

5. Système selon la revendication 1, dans lequel l'unité de planification (4) est configurée pour générer les seconds objectifs et/ou contraintes par addition d'au moins un objectif et/ou une contrainte supplémentaire aux premiers objectifs et contraintes et pour déterminer l'au moins un objectif et/ou une contrainte supplémentaire sur la base des première et seconde entrées d'utilisateur.

6. Système selon la revendication 5, dans lequel l'au moins un objectif et/ou une contrainte supplémentaire correspond à une exigence pour la dose délivrée à l'au moins une position (42) spécifiée.

7. Système selon la revendication 6, dans lequel l'unité de planification (4) est configurée pour sélectionner l'exigence à partir d'une exigence de dose maximale et d'une exigence de dose minimale sur la base d'une comparaison entre la valeur de dose spécifiée et une valeur de dose attribuée à l'au moins une position (42) spécifiée en conformité avec la distribution de dose.

8. Système selon la revendication 7, dans lequel l'unité de planification (4) est configurée pour sélectionner l'exigence de dose maximale si la valeur de dose spécifiée est plus grande que la valeur de dose attribuée à l'au moins une position (42) spécifiée en conformité avec la distribution de dose.

9. Système selon la revendication 7, dans lequel l'unité de planification (4) est configurée pour sélectionner l'exigence de dose minimale si la valeur de dose spécifiée est plus petite que la valeur de dose attribuée à l'au moins une position (42) spécifié en conformité avec la distribution de dose.

10. Système selon la revendication 1, dans lequel l'unité de planification (4) est configurée pour générer le premier plan de traitement en minimisant une première fonction de coût contenant une somme pondérée de fonctions d'objectif, chaque fonction d'objectif correspondant à l'un des premiers objectifs.

11. Système selon les revendications 5 et 10, dans lequel l'unité de planification (4) est configurée pour générer le second plan de traitement en minimisant une seconde fonction de coût, la seconde fonction de coût étant générée par addition d'une fonction d'objectif correspondant à l'au moins un objectif supplémentaire à la première fonction de coût.

12. Système selon la revendication 11, dans lequel la fonction d'objectif est additionnée à un poids qui est égal ou supérieur à un maximum des poids des fonctions d'objectif dans la première fonction de coût.

13. Système selon la revendication 1, dans lequel le traitement de thérapie d'ablation comprend un traitement de curiethérapie temporelle et dans lequel le plan de traitement spécifie une durée de temporisation pour le traitement de curiethérapie temporelle.

14. Procédé pour générer un plan de traitement pour un traitement de thérapie d'ablation d'une structure cible au sein d'une région de traitement, le plan de traitement étant généré sur la base d'objectifs et/ou contraintes d'optimisation représentant des buts de traitement, le procédé comprenant :

la visualisation pour un utilisateur d'une distribution de dose dans la région de traitement, la distribution de dose correspondant à un premier plan de traitement généré sur la base de premiers objectifs et/ou contraintes,
la réception d'une première entrée d'utilisateur spécifiant une valeur de dose dans la distribution de dose et d'une seconde entrée d'utilisateur spécifiant au moins une position (42) dans la région de traitement en tant que position cible pour la valeur de dose,
la détermination de seconds objectifs et/ou contraintes sur la base des premiers objectifs et/ou contraintes et des première et seconde entrées d'utilisateur, et
la génération d'un second plan de traitement sur la base des seconds objectifs et/ou contraintes.

15. Programme d'ordinateur pouvant être exécuté dans une unité de traitement d'un système pour générer un plan de traitement pour un traitement de thérapie d'ablation, le programme d'ordinateur comprenant un moyen de code de programme pour amener l'unité de traitement à réaliser un procédé pour générer un plan de traitement pour un patient selon la revendication 14 lorsque le programme d'ordinateur est exécuté dans l'unité de traitement.

FIG. 1

FIG. 2

FIG. 3

**FIG. 4A**

**FIG. 4B**

FIG. 5

**EP 3 532 163 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070201614 A